# EUROPÄISCHE PATENTSCHRIFT

(11) **EP 2 465 411 B1**
(45) Veröffentlichungstag und Bekanntmachung des Hinweises auf die Patenterteilung: **09.03.2016**
(21) Anmeldenummer: 11189554.6
(22) Anmeldetag: 17.11.2011
(51) Int. Cl.: A61B 1/07, A61B 1/06, A61B 1/00, G02B 23/24

(54) **Endoskop mit einstellbarer Blickrichtung**
Endoscope with adjustable view angle
Endoscope à direction d'observation réglable

(30) Priorität: 16.12.2010 DE 102010063230
(43) Veröffentlichungstag der Anmeldung: 20.06.2012
(73) Patentinhaber: Karl Storz GmbH & Co. KG, 78532 Tuttlingen (DE)
(72) Erfinder: Dahmen, Jan, 78532 Tuttlingen (DE); Lei, Fang, Dr., 78532 Tuttlingen (DE)

(56) Entgegenhaltungen:
- EP-A1- 2 415 391
- DE-T2- 60 015 375
- US-A- 3 818 902
- US-A- 3 896 793

## Beschreibung

Die vorliegende Erfindung bezieht sich auf ein Endoskop mit einstellbarer Blickrichtung.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Die Beobachtung eines Gegenstands in einem Hohlraum mittels eines Endoskops setzt in der Regel eine Beleuchtung des Gegenstands voraus. Dazu weist ein Endoskop beispielsweise Lichtwellenleiter, insbesondere Glasfasern, auf, mittels derer Beleuchtungslicht vom proximalen Ende des Endoskops entlang des Schafts zum distalen Ende des Endoskops übertragen wird. Lichtaustrittsflächen der Lichtwellenleiter am distalen Ende des Endoskops sind so angeordnet und ausgebildet, dass das gesamte Sichtfeld bzw. Blickfeld ausreichend ausgeleuchtet wird.

Bei einem Endoskop mit einstellbarer Blickrichtung wird das Beleuchtungslicht am distalen Ende des Endoskops im einfachsten Fall so verteilt, dass unabhängig von der jeweils eingestellten Blickrichtung das gesamte Sichtfeld ausgeleuchtet ist. Dies hat jedoch eine Reihe von Nachteilen zur Folge. Insbesondere wird Lichtleistung verschwendet, weil ständig unabhängig von der tatsächlich eingestellten Blickrichtung die gesamten Sichtfelder aller einstellbaren Blickrichtungen ausgeleuchtet werden. Bei einer vorbestimmten erwünschten Helligkeit muss somit insgesamt eine deutlich höhere Lichtleistung zur Verfügung gestellt werden als bei einem Endoskop mit feststehender Blickrichtung.

Ein weiterer Nachteil rührt daher, dass Beleuchtungslicht hoher Intensität Gewebe oder andere Gegenstände photothermisch oder photochemisch schädigen kann. Bei einem Endoskop mit feststehender Blickrichtung fällt ein zu geringer Abstand des distalen Endes des Endoskops zu einem Gegenstand zumindest bei Betrachtung des erfassten Bilds in der Regel auf. Bei Verwendung einer Kamera am Endoskop ist auch eine automatische Warnung von Benutzern möglich, wenn die Helligkeit eines erfassten Bilds eine vorbestimmte Schwelle überschreitet. Bei einem Endoskop mit einstellbarer Blickrichtung fällt jedoch ein Teil des Beleuchtungslichts auf Gegenstände, die außerhalb des Sichtfelds liegen. Eine unerwünschte Annäherung des distalen Endes des Endoskops an diese Gegenstände und eine resultierende Bestrahlung dieser Gegenstände mit einer zu hohen Strahlungsleistung fallen deshalb nicht auf.

Ein weiterer Nachteil besteht darin, dass auch Beleuchtungslicht, das außerhalb des Sichtfelds abgestrahlt wird, von Gegenständen oder opaken Medien gestreut oder reflektiert werden kann. Das reflektierte oder gestreute Beleuchtungslicht kann direkt oder indirekt in den Beobachtungsstrahlengang gelangen. Dadurch können Kontraste und vor allem in dunklen Bildbereichen die Unterscheidbarkeit von Gegenständen verringert werden.

Ein weiterer Nachteil rührt daher, dass die Beleuchtungsstärke bzw. die Intensität des Beleuchtungslichts in der Richtung, in der die Blickrichtung variiert werden kann (oft auch als vertikale Richtung bezeichnet) im Wesentlichen konstant ist, während sie in der dazu senkrechten Richtung (oft auch als horizontale Richtung bezeichnet) in der Regel zum Rand des Sichtfelds hin leicht abnimmt. Von Endoskopen mit feststehender Blickrichtung sind Benutzer jedoch in der Regel eine Beleuchtungsstärke gewohnt, die sowohl in horizontaler als auch in vertikaler Richtung zum Rand des Sichtfelds hin leicht abnimmt. Die in vertikaler Richtung konstante Beleuchtungsstärke kann deshalb als irritierend empfunden werden.

In der DE 600 15 375 T2 ist eine Anordnung mehrerer Prismen beschrieben. Eines der Prismen ist um eine Achse rotierbar, um Beleuchtungslicht in eine einstellbare Blickrichtung zu werfen. Die Erfinder der vorliegenden Erfindung haben jedoch festgestellt, dass bei der beschriebenen Anordnung von Prismen die Verteilung des Beleuchtungslichts innerhalb des Sichtfelds in vielen Fällen unbefriedigend ist. Ferner kann es in der Praxis aufwändig sein, mit den in der DE 600 15 375 T2 beschriebenen Anordnungen gleichzeitig eine weitgehende optische Isolation von Beleuchtungs- und Beobachtungsstrahlengang, eine hohe Lichtstärke im Beobachtungsstrahlengang, geringe Verluste im Beleuchtungsstrahlengang und einen kleinen Schaftdurchmesser zu realisieren.

In US 3,896,793 wird ein ähnliches Endoskop mit ein Verstellmechanismus für ein Beleuchtungssystem oder eine Beobachtungsoptik vorgesehen ist, der dazu dient, die Richtung der optischen Achse des Beleuchtungssytem bzw. der Beobachtungsoptik zu ändern.

Eine Aufgabe der vorliegenden Erfindung besteht darin, ein verbessertes Endoskop mit einstellbarer Blickrichtung zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, am distalen Ende eines Endoskops mit einstellbarer Blickrichtung und entsprechend einstellbarer Beleuchtungsrichtung einen oder mehrere schwenkbare Lichtleiter zum Leiten bzw. Übertragen von Beleuchtungslicht vorzusehen. Dieser oder diese Lichtleiter werden zusammen mit ihrer Lichteintrittsfläche und ihrer Lichtaustrittsfläche um eine Schwenkachse geschwenkt und können zwischen der Lichteintrittsfläche und der Lichtaustrittsfläche jeweils im Rahmen der bei Lichtleitern gegebenen Grenzen beliebig gekrümmt sein, um das Beleuchtungslicht bei geringem Raumbedarf verlustarm zu übertragen.

Ein Endoskop mit einstellbarer Blickrichtung umfasst einen Lichtleiter mit einer Lichteintrittsfläche und einer Lichtaustrittsfläche zum Übertragen von Beleuchtungslicht von der Lichteintrittsfläche zu der Lichtaustrittsfläche des Lichtleiters, wobei der Lichtleiter mit der Lichteintrittsfläche und der Lichtaustrittsfläche relativ zum Endoskop um eine Schwenkachse schwenkbar ist.

Das Endoskop ist insbesondere ausgebildet, um die Blickrichtung und die Beleuchtungsrichtung gemeinsam zu schwenken. Die Blickrichtung ist die Richtung, in der ein Gegenstand bezogen auf das distale Ende des Endoskops liegt, der bei Betrachtung durch das Endoskop in der Mitte eines erfassten Bilds erscheint. Die Beleuchtungsrichtung ist die mittlere Richtung bezogen auf das distale Ende des Endoskops, in die das Beleuchtungslicht abgestrahlt wird. Die Beleuchtungsrichtung und die Blickrichtung entsprechen einander insbesondere. Alternativ sind die Blickrichtung und die Beleuchtungsrichtung unabhängig voneinander einstellbar oder die Beleuchtungsrichtung relativ zur Blickrichtung innerhalb vorbestimmter Grenzen variierbar, um beispielsweise bei kleinen Gegenstandsweiten eine vollständige Ausleuchtung des beobachteten Bereichs zu erreichen.

Die Blickrichtung des Endoskops ist insbesondere um eine Schwenkachse schwenkbar, die senkrecht zur Längsachse des Schafts des Endoskops ist. Die Längsachse des Endoskops ist insbesondere die Längsachse des Schafts. Im Fall eines starren geraden Schafts ist die Längsachse des Schafts die Gerade, auf der die Mittelpunkte der Querschnittsflächen des Schafts liegen. Im Fall eines flexiblen Schafts ist die Längsachse des Endoskops die Längsachse des distalen Endes des Schafts, also die Gerade, auf der Mittelpunkte der Querschnittsflächen des Schafts nahe dessen distalem Ende liegen.

Das vom schwenkbaren Lichtleiter zu übertragende bzw. zu leitende Beleuchtungslicht kann mittels eines feststehenden Lichtleiters von einer am proximalen Ende des Endoskops angeordneten oder über ein Lichtleitkabel mit dem proximalen Ende des Endoskops gekoppelten Lichtquelle zum distalen Ende des Endoskops übertragen werden, um dort in die Lichteintrittsfläche des schwenkbaren Lichtleiters eingekoppelt zu werden. Alternativ überträgt der schwenkbare Lichtleiter von einer am distalen Ende des Endoskops angeordneten Lichtquelle erzeugtes Beleuchtungslicht.

Die Schwenkachse des schwenkbaren Lichtleiters ist insbesondere die Schwenkachse der Blickrichtung oder zu dieser parallel. An der Lichtaustrittsfläche des schwenkbaren Lichtleiters kann eine Linse oder ein anderes den Beleuchtungslichtstrahl formendes optisches Element angeordnet sein. Alternativ kann die Lichtaustrittsfläche des schwenkbaren Lichtleiters selbst gekrümmt sein, um den Beleuchtungslichtstrahl zu formen. Das Endoskop kann einen, zwei oder mehr schwenkbare Lichtleiter umfassen, die gemeinsam oder unabhängig voneinander schwenkbar sein können.

Lichtleiter ermöglichen eine Übertragung von Beleuchtungslicht mit hohem Wirkungsgrad bzw. geringen Verlusten auch entlang einfach oder mehrfach gekrümmter Pfade, die anderenfalls mehrere Reflexionen an reflektierenden Flächen erfordern würden. Die räumlichen Verhältnisse am distalen Ende eines Endoskops mit einstellbarer Blickrichtung sind in der Regel beengt und in erster Linie durch den Beobachtungsstrahlengang und den zur Verfügung stehenden Querschnitt des Schafts eingeschränkt. Der Beobachtungsstrahlengang muss so angelegt sein, dass möglichst viel vom beobachteten Gegenstand reflektiertes, gestreutes oder emittiertes Licht aufgefangen und bei möglichst hoher Abbildungsleistung auf einen lichtempfindlichen Sensor gelenkt oder in eine Stablinsenoptik oder ein geordnetes Bündel von Lichtwellenleitern eingekoppelt wird. Die Verwendung von einem oder mehreren Lichtleitern zur Übertragung von Beleuchtungslicht ermöglicht eine besondere Flexibilität bei der Gestaltung des distalen Endes des Endoskops und des Beobachtungsstrahlengangs.

Bei einem Endoskop, wie es hier beschrieben ist, ist der schwenkbare Lichtleiter insbesondere starr.

Ein starrer bzw. biegesteifer Lichtleiter stellt, zumal bei einer geringen Länge, ein robustes, verschleißarmes Bauelement dar, das auch nach langer Anwendung und zahlreichen Änderungen der Beleuchtungsrichtung zuverlässig und verlustarm Beleuchtungslicht übertragen kann. Insbesondere können Nachteile eines flexiblen Lichtleiters vermieden werden, bei dem der Bruch einzelner Fasern oder andere Folgen von Materialermüdung die Verluste bei der Übertragung von Beleuchtungslicht allmählich erhöhen können.

Der starre schwenkbare Lichtleiter umfasst insbesondere ein Bündel von Lichtwellenleitern, die miteinander verklebt, verschmolzen oder vergossen sind, um das Bündel zu versteifen.

Ein Bündel oder mehrere Bündel von Lichtwellenleitern aus Glas oder Kunststoff ermöglichen eine verlustarme und zuverlässige Übertragung von Beleuchtungslicht und kann vor dem teilweisen oder vollständigen Verkleben, Verschmelzen oder Vergießen in eine nahezu beliebige Form gebracht werden. Die einzelnen Lichtwellenleiter sind beispielsweise Multimoden- oder Monomoden-Fasern mit einem innerhalb des Querschnittes kontinuierlich oder stufenförmig variierenden Brechungsindex. Durch das Verkleben, Verschmelzen oder Vergießen kann das Bündel eine hohe mechanische Robustheit erhalten. Dadurch können Schäden an einzelnen Lichtwellenleitern verhindert oder zumindest die Wahrscheinlichkeit ihres Auftretens vermindert werden,

Ein Endoskop, wie es hier beschrieben ist, kann eine schwenkbare reflektierende Fläche zum Umlenken von Beleuchtungslicht aus einer Richtung parallel zur Schwenkachse des schwenkbaren Lichtleiters zur Lichteintrittsfläche des schwenkbaren Lichtleiters umfassen, wobei die schwenkbare reflektierende Fläche dazu vorgesehen und ausgebildet ist, um zusammen mit dem schwenkbaren Lichtleiter geschwenkt zu werden.

Die reflektierende Fläche ist insbesondere eine aufgrund von Totalreflexion oder aufgrund einer Verspiegelung reflektierende Fläche eines Prismas oder eines anderen transparenten Körpers, in dem das Beleuchtungslicht sich zwischen einer Lichteintrittsfläche und einer Lichtaustrittsfläche ausbreitet. Die Flächennormale der Lichteintrittsfläche des schwenkbaren Lichtleiters ist insbesondere senkrecht oder im Wesentlichen senkrecht zur Schwenkachse des Lichtleiters. Durch die Umlenkung des Beleuchtungslichts durch die reflektierende Fläche kann der schwenkbare Lichtleiter eine gerade oder im Wesentlichen gerade Gestalt oder eine vergleichsweise geringe Krümmung aufweisen. Die reflektierende Fläche kann so ausgebildet sein, dass sie einen geringen Bauraum beansprucht.

Die schwenkbare reflektierende Fläche ist insbesondere eine reflektierende Fläche eines schwenkbaren Prismas mit einer Lichteintrittsfläche und einer Lichtaustrittsfläche, wobei die Lichteintrittsfläche des Lichtleiters mit der Lichtaustrittsfläche des Prismas gefügt ist.

Ein schwenkbares Prisma im Sinne der vorliegenden Erfindung ist ein schwenkbarer transparenter Körper, der die Gestalt eines Prismas im strengen Sinn der Geometrie aufweist, dessen Oberfläche also zwei gleiche und parallele Polygone umfasst, deren entsprechende Ecken durch parallele Seitenkanten miteinander verbunden sind. Darüber hinaus wird der Begriff Prisma in der Optik für transparente Körper mit einer Lichteintrittsfläche und einer Lichtaustrittsfläche, die nicht parallel zueinander sind, verwendet.

Bei der vorliegenden Erfindung kann der transparente Körper noch allgemeiner eine ebene oder gekrümmte Lichteintrittsfläche, eine ebene oder gekrümmte reflektierende Fläche und eine ebene oder gekrümmte Lichtaustrittsfläche zum Umlenken von Beleuchtungslicht aufweisen. Eine gekrümmte Lichteintrittsfläche, eine gekrümmte reflektierende Fläche und/oder eine gekrümmte Lichtaustrittsfläche kann eine Veränderung der Divergenz oder Konvergenz des Beleuchtungslichts oder eine andere Strahlformung bewirken.

Die Lichteintrittsfläche des schwenkbaren Lichtleiters und die Lichtaustrittsfläche des transparenten Körpers sind insbesondere verklebt oder verschweißt.

Ein Prisma oder ein anderer transparenter Körper kann ein kompaktes und robustes optisches Element zur Umlenkung von Beleuchtungslicht darstellen.

Bei einem Endoskop, wie es hier beschrieben ist, kann der schwenkbare Lichtleiter eine Krümmung aufweisen.

Insbesondere eine Krümmung um einen Winkel von 90 Grad oder im Wesentlichen 90 Grad kann eine Alternative zu der oben genannten schwenkbaren reflektierenden Fläche darstellen, um Beleuchtungslicht aus einer Richtung parallel zur Schwenkachse des schwenkbaren Lichtleiters in die Beleuchtungsrichtung umzulenken. Dazu weist der Lichtleiter insbesondere an seiner Lichteintrittsfläche eine Richtung parallel zur Schwenkachse und an seiner Lichtaustrittsfläche eine Richtung parallel zur Beleuchtungsrichtung auf. Bei einem Lichtleiter, der bei Veränderung der Beleuchtungsrichtung weder elastisch noch plastisch verformt wird, können auch kleine Krümmungsradien bei geringen Übertragungsverlusten und hoher Zuverlässigkeit realisierbar sein.

Der schwenkbare Lichtleiter ist an der Lichteintrittsfläche des schwenkbaren Lichtleiters insbesondere in einer Richtung parallel zu einer Schwenkachse des schwenkbaren Lichtleiters angeordnet.

Bei einem Endoskop, bei dem der schwenkbare Lichtleiter an der Lichteintrittsfläche des schwenkbaren Lichtleiters in einer Richtung parallel zur Schwenkachse des schwenkbaren Lichtleiters angeordnet ist, ist die Lichteintrittsfläche des schwenkbaren Lichtleiters insbesondere kreisförmig oder kreisringförmig.

Eine kreisförmige oder kreisringförmige und insbesondere zur Schwenkachse des schwenkbaren Lichtleiters symmetrische Lichteintrittsfläche des schwenkbaren Lichtleiters ermöglicht eine von der Position des schwenkbaren Lichtleiters unabhängige Einkopplung von Beleuchtungslicht in den schwenkbaren Lichtleiter. Dadurch kann die Ausleuchtung des Sichtfelds von der Blickrichtung unabhängig sein. Dies gilt insbesondere, wenn der kreisförmigen oder kreisringförmigen Lichteintrittsfläche des schwenkbaren Lichtleiters eine entsprechend ausgebildete und angeordnete Lichtaustrittsfläche einer Lichtquelle oder eines feststehenden Lichtleiters gegenüberliegt.

Eine kreisringförmige Ausgestaltung der Lichteintrittsfläche des schwenkbaren Lichtleiters und der Lichtaustrittsfläche der Lichtquelle bzw. des feststehenden Lichtleiters ermöglicht beispielsweise eine Anordnung eines Lagers, einer Welle oder eines Abschnitts des Beobachtungsstrahlungsgangs innerhalb der kreisringförmigen Lichteintritts- bzw. Lichtaustrittsflächen und damit eine besonders raumsparende Anordnung.

Ein Endoskop, wie es hier beschrieben ist, kann eine feststehende reflektierende Fläche zum Umlenken von Beleuchtungslicht in eine Richtung parallel zur Schwenkachse des schwenkbaren Lichtleiters umfassen.

Die feststehende reflektierende Fläche ist insbesondere eine reflektierende Fläche eines Spiegels oder eines Prismas oder eines anderen transparenten Körpers, die aufgrund von Totalreflexion oder aufgrund einer reflektierenden Beschichtung reflektiert. Mittels einer feststehenden reflektierenden Fläche wird beispielsweise Beleuchtungslicht, das mittels eines feststehenden Lichtleiters zum distalen Ende des Endoskops übertragen wird, in eine Richtung parallel zur Schwenkachse des schwenkbaren Lichtleiters umgelenkt. Eine feststehende reflektierende Fläche kann eine besonders platzsparende Umlenkung von Beleuchtungslicht ermöglichen.

Ein Endoskop, wie es hier beschrieben ist, kann einen feststehenden Lichtleiter zum Übertragen von Beleuchtungslicht zum distalen Ende des Endoskops umfassen, wobei der feststehende Lichtleiter nahe seiner Lichtaustrittsfläche eine Krümmung aufweist.

Die Flächennormale der Lichtaustrittsfläche des feststehenden Lichtleiters ist insbesondere parallel zur Schwenkachse des schwenkbaren Lichtleiters. Die Lichtaustrittsfläche des feststehenden Lichtleiters ist insbesondere gegenüber einer hinsichtlich ihrer Gestalt, ihrer Anordnung und ihrer Ausrichtung entsprechenden Lichteintrittsfläche des schwenkbaren Lichtleiters angeordnet. Der feststehende Lichtleiter ist nahe seiner Lichtaustrittsfläche insbesondere um 90 Grad gekrümmt.

Der an seinem distalen Ende gekrümmte feststehende Lichtleiter kann eine konstruktiv besonders einfache und verlustarme Übertragung von Beleuchtungslicht zu dem schwenkbaren Lichtleiter ermöglichen. Da der feststehende Lichtleiter bei einer Veränderung der Blickrichtung und der Beleuchtungsrichtung nicht verformt wird, sondern lediglich der schwenkbare Lichtleiter gegenüber dem feststehenden Lichtleiter geschwenkt wird, ist ein kleiner Krümmungsradius am feststehenden Lichtleiter realisierbar.

Ein Endoskop, wie es hier beschrieben ist, kann einen um die Schwenkachse der Blickrichtung schwenkbaren Lichtschacht zur optischen Trennung des Beobachtungsstrahlengangs vom Beleuchtungsstrahlengang aufweisen, wobei die Lichtaustrittsfläche des schwenkbaren Lichtleiters außen an dem Lichtschacht angeordnet ist.

Der Lichtschacht weist insbesondere die Form eines Kegelstumpfes mit kreisförmiger oder nicht kreisförmiger Grundfläche, insbesondere eines Konus oder eines Pyramidenstumpfes, mit kreisförmigem, elliptischem oder rechteckigem Rand auf. Die Lichtaustrittsfläche des schwenkbaren Lichtleiters ist insbesondere in einem oder mehren jeweils im Wesentlichen geraden oder gebogenen streifenförmigen Teilen außen am Rand des Lichtschachts angeordnet.

Ein mit der Blickrichtung schwenkender Lichtschacht ermöglicht eine weitgehende oder vollständige optische Isolation bzw. Trennung des Beobachtungsstrahlengangs vom Beleuchtungsstrahlengang. Eine Anordnung der Lichtaustrittsfläche in einem oder mehreren Teilen außen am Lichtschacht gewährleistet auf konstruktiv einfache Weise, dass die Lichtaustrittsfläche und damit auch die Beleuchtungsrichtung mit der Blickrichtung schwenkt. Gleichzeitig kann die Lichtaustrittsfläche besonders nah am Beobachtungsstrahlengang angeordnet sein, um störende Schatten zu minimieren.

Bei einem Endoskop, wie es hier beschrieben ist, kann an der Lichtaustrittsfläche des schwenkbaren Lichtleiters eine Lichtaustrittseinrichtung angeordnet sein. Die Lichtaustrittseinrichtung kann eine oder mehrere Linsen, Spiegel, Prismen oder andere optische Elemente zur Formung eines am distalen Ende aus dem Lichtleiter ausgekoppelten Beleuchtungslichtbündels umfassen. Alternativ ist die Lichtaustrittsfläche des schwenkbaren Lichtleiters zur Formung des ausgekoppelten Beleuchtungslichtbündels ausgebildet. Im einfachsten Fall ist die Lichtaustrittsfläche des schwenkbaren Lichtbündels eben, und die Divergenz des austretenden Beleuchtungslichtbündels wird aufgrund der Übertragungseigenschaften des Lichtleiters bereits bei der Einkopplung des Beleuchtungslichts in den schwenkbaren Lichtleiter festgelegt.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Darstellung des distalen Endes einer Ausführungsform des Endoskops aus Figur 1;
- Figur 3: eine weitere schematische Darstellung des distalen Endes aus Figur 2;
- Figur 4: schematische Darstellungen von drei Varianten der Ausführungsform aus den Figuren 2 und 3;
- Figur 5: schematische Darstellungen von zwei Varianten der Ausführungsform aus den Figuren 2 und 3;
- Figur 6: eine schematische Darstellung des distalen Endes einer weiteren Ausführungsform des Endoskops aus Figur 1;
- Figur 7: eine schematische Darstellung des distalen Endes einer weiteren Ausführungsform des Endoskops aus Figur 1;
- Figur 8: eine schematische Darstellung des distalen Endes einer weiteren Ausführungsform des Endoskops aus Figur 1;
- Figur 9: eine schematische Darstellung des distalen Endes einer weiteren Ausführungsform des Endoskops aus Figur 1
- Figur 10: eine schematische Darstellung einer Einrichtung zum Schwenken;
- Figur 11: eine weitere schematische Darstellung der Einrichtung aus Figur 10.

### Beschreibung von Ausführungsformen

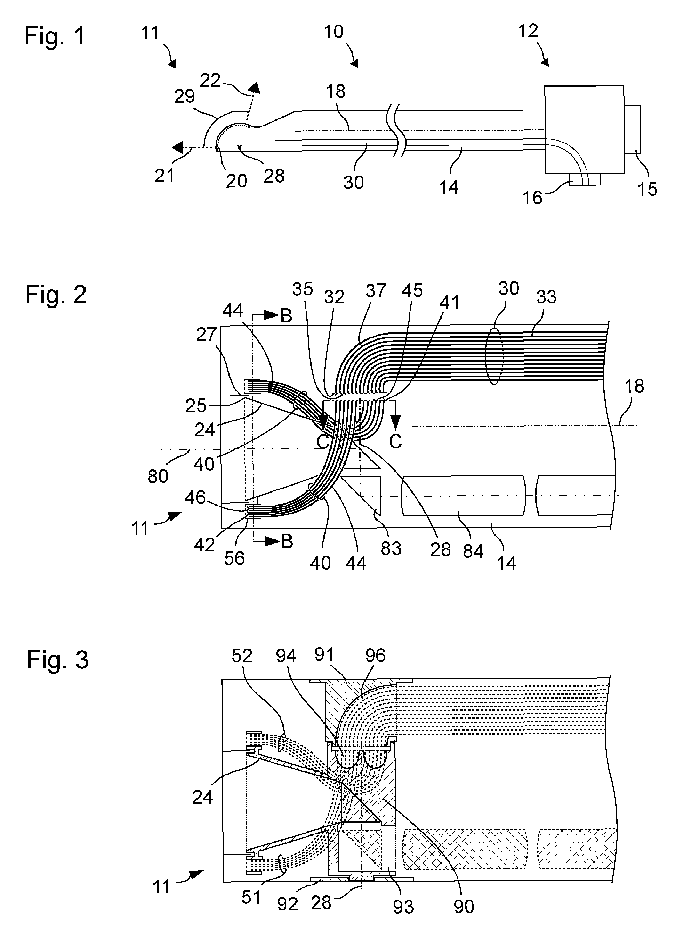

Figur 1 zeigt eine schematische Darstellung eines Endoskops 10 mit einem distalen Ende 11, einem proximalen Ende 12 und einem starren Schaft 14, der sich vom distalen Ende 11 bis zum proximalen Ende 12 erstreckt. Alternativ ist der Schaft 14 flexibel oder teilweise flexibel. Der Querschnitt des Schafts 14 oder zumindest die äußere Kontur des Querschnitts des Schafts 14 ist zwischen dem distalen Ende 11 und dem proximalen Ende 12 konstant oder im Wesentlichen konstant. Insbesondere ist die Kontur des Querschnitts des Schafts 14 kreisförmig oder elliptisch. In diesem Fall ist die in Figur 1 dargestellte Längsachse 18 des Endoskops 10 die Symmetrieachse der Mantelfläche des Schafts 14 zwischen dem distalen Ende 11 und dem proximalen Ende 12. Bei einer zylindrischen Mantelfläche des Schafts 14 ist die Längsachse 18 auch die Menge der Mittelpunkte oder Flächenschwerpunkte der Querschnitte des Schafts 14 zwischen dem distalen Ende 11 und dem proximalen Ende 12. Bei einer kreiszylindrischen Mantelfläche des Schafts 14 ist die Längsachse 18 auch die Symmetrieachse der Mantelfläche.

Am distalen Ende 11 weicht die Gestalt des Schafts 14 von der Zylindersymmetrie ab, wie dies beispielhaft in Figur 1 dargestellt ist. Insbesondere weist der Schaft 14 am distalen Ende 11 eine Öffnung auf, die durch ein transparentes Fensterbauteil 20 mit einer gewölbten Oberfläche verschlossen ist. Insbesondere verschließt das Fensterbauteil 20 die Öffnung hermetisch dicht. Die Oberfläche des Fensterbauteils 20 hat beispielsweise die Gestalt eines Ausschnitts eines Kreiszylindermantels, wobei die Symmetrieachse des Kreiszylinders senkrecht zur Längsachse 18 des Endoskops 10 und zur Zeichenebene der Figur 1 ist. Alternativ hat die Oberfläche des transparenten Fensterbauteils 20 die Gestalt eines Ausschnitts einer Kugeloberfläche oder eines rotationssymmetrischen oder nicht rotationssymmetrischen Ellipsoids.

Am distalen Ende 11 des Endoskops 10 sind im Schaft 14 optische Einrichtungen angeordnet, die nachfolgend mit Bezug auf die Figuren 2 bis 9 teilweise beschrieben werden. Diese optischen Einrichtungen ermöglichen eine Variation der Blickrichtung des Endoskops zwischen einer ersten extremen Blickrichtung 21 und einer zweiten extremen Blickrichtung 22. Die Blickrichtung ist zwischen den beiden extremen Blickrichtungen 21, 22 um eine Schwenkachse 28 schwenkbar, die senkrecht zur Zeichenebene der Figur 1 ist. Die Blickrichtung ist jeweils die Richtung bezogen auf das distale Ende 11 des Endoskops 10, in der ein Gegenstand liegt, der in der Mitte eines mittels des Endoskops 10 erfassten Bilds erscheint.

Bei dem in Figur 1 dargestellten Beispiel ist die erste extreme Blickrichtung 21 parallel oder im Wesentlichen parallel zur Längsachse 18 des Endoskops 10. Zwischen den extremen Blickrichtungen 21, 22 liegt ein Winkelbereich 29, der bei dem dargestellten Beispiel ca. 120 Grad umfasst. Innerhalb dieses Winkelbereichs ist die Blickrichtung des Endoskops 10 insbesondere kontinuierlich verstellbar bzw. einstellbar.

Am proximalen Ende 12 weist das Endoskop 10 eine erste Kupplung 15 zum optischen Koppeln des Endoskops 10 mit einer Kamera oder ein Okular sowie eine zweite Kupplung 16 zum Koppeln des Endoskops 10 mit einer Lichtquelle über ein Lichtleitkabel auf. Von der zweiten Kupplung 16 führen ein oder mehrere Lichtleiter 30 durch den Schaft 14 bis zum distalen Ende 11 des Endoskops 10. Von einer Lichtquelle erzeugtes Beleuchtungslicht kann über ein Lichtleitkabel, die zweite Kupplung 16 und den oder die Lichtleiter 30 zum distalen Ende 11 des Endoskops 10 übertragen werden.

Die Figuren 2 und 3 zeigen schematische Darstellungen einer Ausführungsform des distalen Endes 11 des oben anhand der Figur 1 dargestellten Endoskops 10. Die Figur 2 zeigt eine schematische Darstellung, deren Zeichenebene senkrecht zur Zeichenebene der Figur 1 und parallel zur Längsachse 18 des Endoskops 10 und zur Schwenkachse 28 der Blickrichtung ist.

Der bereits in Figur 1 angedeutete Lichtleiter 30 weist am distalen Ende 11 des Endoskops 10 eine Lichtaustrittsfläche 32 auf. Der Lichtleiter 30 umfasst ein Bündel von Lichtwellenleitern 33, deren Lichtaustrittsflächen 35 in einer Ebene angeordnet sind und gemeinsam die Lichtaustrittsfläche 32 des Lichtleiters 30 bilden. Unmittelbar lichtstromaufwärts der Lichtaustrittsfläche 32 weist der Lichtleiter 30 eine Krümmung 37 um einen Winkel von ca. 90 Grad auf. Lichtstromaufwärts der Krümmung 37 verläuft der Lichtleiter 30 parallel oder im Wesentlichen parallel zur Längsachse 18 des Schafts 14 des Endoskops 10. An der Lichtaustrittsfläche 32 des feststehenden Lichtleiters 30 verlaufen die Lichtwellenleiter 33 und damit der gesamte Lichtleiter 30 im Wesentlichen parallel zur Schwenkachse 28 der Blickrichtung. Auch die Flächennormalen der Lichtaustrittsfläche 32 des Lichtleiters 30 und der Lichtaustrittsflächen 35 der Lichtwellenleiter 33 sind parallel oder im Wesentlichen parallel zur Schwenkachse 28 der Blickrichtung.

Am distalen Ende 11 des Endoskops 10 sind ferner schwenkbare Lichtleiter 40 angeordnet. Jeder schwenkbare Lichtleiter 40 umfasst eine Lichteintrittsfläche 41, die der Lichtaustrittsfläche 32 des feststehenden Lichtleiters 30 gegenüberliegt, und eine Lichtaustrittsfläche 42. Jeder schwenkbare Lichtleiter 40 umfasst ein Bündel von Lichtwellenleitern 44. Lichteintrittsflächen 45 der Lichtwellenleiter 44 bilden zusammen die Lichteintrittsfläche 41 des schwenkbaren Lichtleiters, Lichtaustrittsflächen 46 der Lichtwellenleiter bilden zusammen die Lichtaustrittsfläche 42 des schwenkbaren Lichtleiters 40.

Am distalen Ende 11 des Endoskops 10 ist ferner das lichtstromaufwärtige Ende eines Beobachtungsstrahlengangs 80 angeordnet, dessen optische Achse in Figur 2 durch eine strichpunktierte Linie angedeutet ist. Der Beobachtungsstrahlengang 80 umfasst lichtstromabwärts des in Figur 1 dargestellten Fensterbauteils 20 ein schwenkbares Prisma 82, ein feststehendes Prisma 83 und eine Stablinsenanordnung 84 zum Übertragen von Licht, das von einem zu beobachtenden Gegenstand ausgeht, zum proximalen Ende 12 des Endoskops 10. Zwischen dem in Figur 2 nicht dargestellten Fensterbauteil 20 und dem schwenkbaren Prisma 82 ist ein Lichtschacht 24 angeordnet. Die Wand des Lichtschachts 24 weist ein lichtabsorbierendes Material auf. Der Lichtschacht 24 weist beispielsweise die Gestalt eines Kegelstumpfes, insbesondere eines Pyramidenstumpfes auf. Nahe dem lichtstromaufwärtigen Rand 25 des Lichtschachts 24 sind eine oder mehrere Fassungen 56 für die Lichtaustrittsflächen 42 der schwenkbaren Lichtleiter 44 angeordnet. In einen Zwischenraum zwischen dem Rand 25 des Lichtschachts 24 und der oder den Fassungen 56 greift eine Blende 27 ein.

Die schwenkbaren Lichtleiter 40 sind zwischen ihren Lichteintrittsflächen 41 und ihren Lichtaustrittsfläche 42 gekrümmt. Insbesondere umfassen die schwenkbaren Lichtleiter 40 jeweils mindestens eine Biegung um mindestens 90 Grad. An ihren Lichteintrittsflächen 41 verlaufen die Lichtleiter 40 im Wesentlichen parallel zur Schwenkachse 28 der Blickrichtung. An ihren Lichtaustrittsflächen 42 verlaufen die schwenkbaren Lichtleiter 40 im Wesentlichen parallel zur Blickrichtung und damit senkrecht zur Schwenkachse 28. Das in Figur 2 dargestellte Beispiel umfasst zwei Lichtleiter, deren Lichtaustrittsflächen 42 an gegenüberliegenden Seiten des Lichtschachts 24 angeordnet sind.

Die schwenkbaren Lichtleiter 40, das schwenkbare Prisma 82, der Lichtschacht 24 und die Fassung oder die Fassungen 56 sind starr miteinander verbunden und gemeinsam um die Schwenkachse 28 der Blickrichtung schwenkbar. Dadurch ist gewährleistet, dass die im Wesentlichen durch die Anordnung der Lichtaustrittsflächen 42 der schwenkbaren Lichtleiter 40 gegebene Beleuchtungsrichtung und die im Wesentlichen durch die Position des schwenkbaren Prismas 82 gegebene Blickrichtung gemeinsam verändert werden und insbesondere jederzeit gleich sind.

Der Lichtschacht 24 und die insbesondere kreisbogenförmigen Blenden 27 verhindern weitgehend oder vollständig eine Einkopplung von aus den Lichtaustrittsflächen 42 der schwenkbaren Lichtleiter 40 austretendem Beleuchtungslicht in den Beobachtungsstrahlengang. Die Lichtaustrittsfläche 32 des feststehenden Lichtleiters 30 und die Lichteintrittsflächen 41 der schwenkbaren Lichtleiter 40 sind jeweils im Wesentlichen eben und liegen einander mit kleinem Abstand gegenüber. Der Abstand zwischen der Lichtaustrittsfläche 32 des feststehenden Lichtleiters 30 und der Lichteintrittsfläche 41 des schwenkbaren Lichtleiters ist insbesondere wesentlich kleiner als in Figur 2 schematisch angedeutet. Da die Flächennormalen der Lichtaustrittsfläche 32 des feststehenden Lichtleiters 30 und der Lichteintrittsflächen 41 der schwenkbaren Lichtleiter 40 jeweils parallel zur Schwenkachse 28 der Blickrichtung sind, gilt dies unabhängig oder weitgehend unabhängig von der momentan eingestellten Beleuchtungs- und Blickrichtung.

Die Gestaltung des feststehenden Lichtleiters 30 und der schwenkbaren Lichtleiter 40 jeweils aus einem Bündel von Lichtwellenleitern 33, 44 ermöglicht kleine Krümmungsradien und eine raumsparende Anordnung, insbesondere der schwenkbaren Lichtleiter 40.

Dadurch können ein kleinerer Querschnitt des Schafts 14 und/oder größere Querschnitte im Beobachtungsstrahlengang 80 realisierbar sein.

Figur 3 zeigt eine schematische Schnittdarstellung der oben anhand der Figur 2 dargestellten Ausführungsform. Die Schnittebene der Figur 3 ist parallel zur Zeichenebene der Figur 2. Die bereits oben anhand der Figur 2 dargestellten feststehenden und schwenkbaren Lichtleiter 30, 40 und die Elemente 82, 83, 84 des Beobachtungsstrahlengangs 80 sind lediglich in gestrichelten Linien angedeutet. Stattdessen ist eine in Figur 2 nicht dargestellte schwenkbare Beleuchtungs- und Beobachtungseinrichtung 90 gezeigt.

Die schwenkbare Beleuchtungs- und Beobachtungseinrichtung 90 ist in zwei an gegenüberliegenden Seiten des Schafts 14 des Endoskops 10 befestigten Lagern 91, 92 um die Schwenkachse 28 der Blickrichtung schwenkbar gelagert. Die schwenkbare Beleuchtungs- und Beobachtungseinrichtung 90 umfasst den Lichtschacht 24 bzw. dessen Wand und die am Rand 25 des Lichtschachts 24 angeordneten Fassungen 56. Die schwenkbare Beleuchtungs- und Beobachtungseinrichtung 90 kann einstückig ausgeführt sein.

An oder in der schwenkbaren Beleuchtungs- und Beobachtungseinrichtung 90 ist das schwenkbare Prisma 82 des Beobachtungsstrahlengangs 80 gehalten. Ferner umfasst die schwenkbare Beleuchtungs- und Beobachtungseinrichtung 90 Kanäle 94, in denen die schwenkbaren Lichtleiter 40 teilweise verlaufen. Diese Kanäle 94 sind durch die Schnittebene der Figur 3 angeschnitten. In einer Ausnehmung 93 der schwenkbaren Beleuchtungs- und Beobachtungseinrichtung 90 ist das feststehende Prisma 83 des Beobachtungsstrahlengangs 80 angeordnet, das nicht mit der schwenkbaren Beleuchtungs- und Beobachtungseinrichtung 90 bewegt wird.

Im ersten Lager 91 ist ein gekrümmter Kanal 96 vorgesehen, in dem der feststehende Lichtleiter 30 teilweise angeordnet ist. Der feststehende Lichtleiter 30 kann mit dem ersten Lager 91 verklebt oder vergossen oder auf andere Weise gefügt sein. Das erste Lager 91 und das zweite Lager 92 können mit der Wand des Schafts 14 des Endoskops 10 einstückig ausgeführt, verschweißt, verschraubt oder verklebt sein.

Die schwenkbare Beleuchtungs- und Beobachtungseinrichtung 90 bildet zusammen mit den schwenkbaren Lichtleitern 40 und dem schwenkbaren Prisma 82 ein kompaktes und robustes Bauteil, das außerhalb des Endoskops 10 vormontiert und dann in dieses eingesetzt werden kann. Da die schwenkbaren Lichtleiter 40 beim Schwenken der Beleuchtungs- und Blickrichtung um die Schwenkachse 28 nicht verformt werden, ist das Risiko einer Beschädigung, insbesondere eines Bruchs einzelner Lichtwellenleiter 44, gering. Zur weiteren Verbesserung der Robustheit sind die einzelnen Lichtwellenleiter 44 der schwenkbaren Lichtleiter 40 untereinander und/oder mit der schwenkbaren Beleuchtungs- und Beobachtungseinrichtung 90 insbesondere verklebt, verschmolzen oder vergossen.

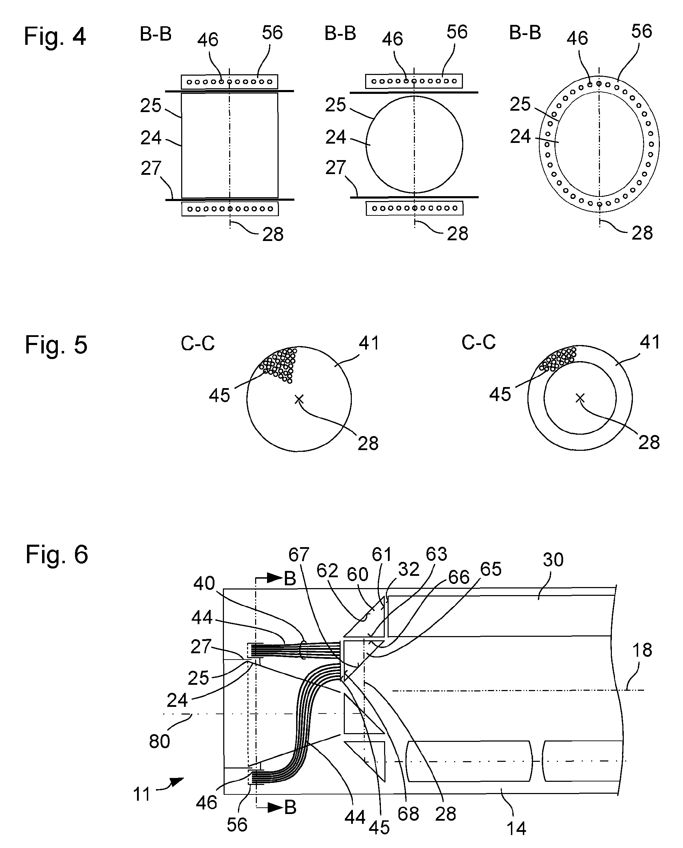

Figur 4 zeigt schematische Schnittdarstellungen dreier Varianten des Lichtschachts 24 und der Fassung bzw. der Fassungen 56. Die Lage der jeweils dargestellten Schnittebene B-B ist in Figur 2 angedeutet. Die Schnittebene B-B ist senkrecht zu den Zeichenebenen der Figuren 1 bis 3, senkrecht zur Längsachse 18 des Schafts 14 des Endoskops 10 und parallel zur Schwenkachse 28 der Blick- und Beobachtungsrichtung. In Figur 4 ist jeweils auch die Position der Schwenkachse 28 der Beleuchtungs- und Blickrichtung angedeutet, die jedoch nicht in den dargestellten Schnittebenen liegt.

Bei dem in Figur 4 links dargestellten Beispiel weisen der Lichtschacht 24 die Gestalt eines Kegelstumpfes mit rechteckiger Grundfläche und der Rand 25 entsprechend die Form eines Rechtecks auf. An zwei gegenüberliegenden Seiten des durch den Rand 25 gebildeten Rechtecks sind jeweils eine streifenförmige bzw. schmale und längliche Fassung 56 für die Enden und die Lichtaustrittsflächen 46 der Lichtwellenleiter 44 angeordnet. Zwischen dem Rand 25 des Lichtschachts 24 und der Fassung 56 ist jeweils eine der bereits in den Figuren 2 und 3 dargestellten Blenden 27 angeordnet. Anstelle zweier Fassungen 56 an einander gegenüberliegenden Seiten des durch den Rand 25 gebildeten Rechtecks können beispielsweise eine einzige gerade streifenförmige Fassung an einer Seite, an jeder Seite eine gerade streifenförmige Fassung oder eine den Lichtschacht 24 umschließende rahmenförmige Fassung vorgesehen sein.

Das in Figur 4 in der Mitte dargestellte Beispiel unterscheidet sich von dem links dargestellten Beispiel dadurch, dass der Lichtschacht die Gestalt eines Kegelstumpfes mit einer kreisförmigen Grundfläche aufweist. Entsprechend weist der Rand 25 des Lichtschachts 24 eine Kreisform auf.

In Figur 4 rechts ist ein Beispiel dargestellt, bei dem der Lichtschacht 24 die Gestalt eines Kegelstumpfes mit elliptischer Grundfläche und der Rand 25 des Lichtschachts 24 entsprechend die Form einer Ellipse aufweisen. Die Fassung 56 umschließt in Form einer Ellipse den gesamten Lichtschacht 24 bzw. dessen Rand 25. Eine zwischen dem Rand 25 des Lichtschachts 24 und die Fassung 56 eingreifende Blende wie bei den in Figur 4 links und in der Mitte dargestellten Beispielen und wie in den Figuren 2 und 3 dargestellt, ist bei dem in Figur 4 rechts dargestelltem Beispiel nicht vorgesehen. Stattdessen ist beispielsweise der Rand 25 bis zu einem möglichst geringen Abstand an das oben in Figur 1 dargestellte Fensterbauteil 20 herangeführt.

Anstelle einer kegelstumpfförmigen Gestalt kann der Lichtschacht 24 eine andere Gestalt aufweisen. Beispielsweise kann der Lichtschacht 24 an seiner lichtstromaufwärtigen Seite einen rechteckigen und an seiner lichtstromabwärtigen Seite einen quadratischen Querschnitt oder an seiner lichtstromaufwärtigen Seite einen elliptischen und an seiner lichtstromabwärtigen Seite einen rechteckigen Querschnitt aufweisen.

Figur 5 zeigt eine schematische Schnittdarstellung zweier Varianten der Lichteintrittsfläche 41 des oder der schwenkbaren Lichtleiter 40. Die Lage der dargestellten Schnittebene C-C ist in Figur 2 angedeutet. Die Schnittebene C-C enthält jeweils die Lichtaustrittsfläche 41. In Figur 5 ist ferner jeweils die Lage der zu der Schnittebene C-C und zu der Lichteintrittsfläche 41 senkrechten Schwenkachse 28 der Beleuchtungs- und Blickrichtung angedeutet. Von den Lichteintrittsflächen 45 der Lichtwellenleiter 44 ist nur beispielhaft ein Teil angedeutet.

Bei dem in Figur 5 links dargestellten Beispiel ist die Lichteintrittsfläche 41 kreisförmig, bei dem in Figur 5 rechts dargestellten Beispiel ist die Lichteintrittsfläche 41 kreisringförmig. In beiden Fällen wird die Lichteintrittsfläche 41, wie bereits oben in Zusammenhang mit der Figur 2 erwähnt, durch die Lichteintrittsflächen 45 der einzelnen Lichtwellenleiter gebildet. Bei der in Figur 5 rechts dargestellten kreisringförmigen Gestalt der Lichteintrittsfläche 41 kann abweichend von der Darstellung in Figur 3 die schwenkbare Beleuchtungs- und Beobachtungseinrichtung 90 innerhalb der Lichteintrittsfläche 41 gelagert sein.

Figur 6 zeigt eine schematische Darstellung des distalen Endes 11 einer weiteren Ausführungsform des Endoskops 10 aus Figur 1. Die Ausführungsform der Figur 6 ähnelt in einigen Merkmalen der Ausführungsform der Figuren 2 und 3. Abweichend von der Ausführungsform der Figuren 2 und 3 weist der feststehende Lichtleiter 30 keine Krümmung nahe seiner Lichtaustrittsfläche 32 auf. Stattdessen ist der feststehende Lichtleiter 30 insbesondere gerade. Die Lichtaustrittsfläche 32 ist optisch mit einer Lichteintrittsfläche 61 eines feststehenden Prismas 60 gekoppelt. Insbesondere sind die Lichtaustrittsfläche 32 des feststehenden Lichtleiters 30 und die Lichteintrittsfläche 61 des feststehenden Prismas 60 abweichend von der Darstellung in Figur 6 miteinander unmittelbar verschweißt oder durch einen transparenten Kitt oder ein transparentes Lot verbunden.

Das feststehende Prisma 60 weist eine reflektierende Fläche 62 und eine Lichtaustrittsfläche 63 auf. Die Flächennormale der Lichteintrittsfläche 61 des feststehenden Prismas 60 ist parallel oder im Wesentlichen parallel zur Längsachse 18 des Schafts 14 des Endoskops 10. Die Flächennormale der Lichtaustrittsfläche 63 des feststehenden Prismas 60 ist parallel oder im Wesentlichen parallel zur Schwenkachse 28 der Beleuchtungs- und Blickrichtung. Die Flächennormale der reflektierenden Fläche 62 des feststehenden Prismas 60 ist die Winkelhalbierende oder im Wesentlichen die Winkelhalbierende der Flächennormalen der Lichteintrittsfläche 61 und der Flächennormalen der Lichtaustrittsfläche 62 des feststehenden Prismas 60.

Der Lichtaustrittsfläche 63 des feststehenden Prismas 60 gegenüber und parallel zu dieser ist eine Lichteintrittsfläche 66 eines schwenkbaren Prismas 65 angeordnet. Damit ist insbesondere auch die Flächennormale der Lichteintrittsfläche 66 des schwenkbaren Prismas 65 parallel oder im Wesentlichen parallel zur Schwenkachse 28 der Beleuchtungs- und Blickrichtung. Das schwenkbare Prisma 65 weist ferner eine reflektierende Fläche 67 und eine Lichtaustrittsfläche 68 auf. Die Flächennormale der Lichtaustrittsfläche 68 ist senkrecht oder im Wesentlichen senkrecht zur Schwenkachse 28 der Beleuchtungs- und Blickrichtung. Die Flächennormale der reflektierenden Fläche 67 des schwenkbaren Prismas 65 ist die Winkelhalbierende oder im Wesentlichen die Winkelhalbierende der Flächennormalen der Lichteintrittsfläche 66 und der Flächennormalen der Lichtaustrittsfläche 68 des schwenkbaren Prismas 65.

Sowohl die reflektierende Fläche 62 des feststehenden Prismas 60 als auch die reflektierende Fläche 67 des schwenkbaren Prismas 65 kann jeweils durch Totalreflexion oder aufgrund einer reflektierenden Beschichtung reflektieren. Abweichend von der Darstellung in Figur 6 können die Lichteintrittsflächen 61, 66, die reflektierenden Flächen 62, 67 und die Lichtaustrittsflächen 63, 68 des feststehenden Prismas 60 und des schwenkbaren Prismas 65 jeweils gekrümmt sein, um die Konvergenz oder Divergenz des übertragenen Beleuchtungslichtbündels zu verändern.

Die Lichtaustrittsfläche 68 des schwenkbaren Prismas 65 ist den Lichteintrittsflächen 41 von schwenkbaren Lichtleitern 40 gegenüber angeordnet und mit diesen optisch gekoppelt. Insbesondere ist die Lichtaustrittsfläche 68 des schwenkbaren Prismas 65 mit den Lichteintrittsflächen 41 der schwenkbaren Lichtleiter 40 abweichend von der schematischen Darstellung in Figur 6 verschweißt oder durch einen transparenten Kitt oder ein transparentes Lot verbunden. Die lichtstromabwärtigen Enden und die Lichtaustrittsfläche 42 der Lichtleiter 40 sind ähnlich wie bei der Ausführungsform der Figuren 2 und 3 in Fassungen 56 an der Wand eines Lichtschachts 24 angeordnet. Bei dem in Figur 6 gezeigten Beispiel weist einer der beiden schwenkbaren Lichtleiter 40 keine oder nur eine geringe Krümmung auf, der andere Lichtleiter weist eine im Wesentlichen S-förmige Krümmung auf.

Zum Schwenken der Beleuchtungs- und Blickrichtung um die Schwenkachse 28 werden das schwenkbare Prisma 65 und die schwenkbaren Lichtleiter 40 zusammen mit dem schwenkbaren Prisma 82 im Beobachtungsstrahlengang 80, dem Lichtschacht 24 und den Fassungen 56 um die Achse 28 geschwenkt. Dazu kann eine schwenkbare Beleuchtungs- und Beobachtungseinrichtung ähnlich der oben anhand der Figur 3 dargestellten vorgesehen sein. Ähnlich wie die oben anhand der Figuren 2 und 3 dargestellten schwenkbaren Lichtleiter können auch die schwenkbaren Lichtleiter 40 der Ausführungsform der Figur 6 jeweils ein Bündel von Lichtwellenleitern 44 umfassen. Zur Verbesserung der mechanischen Robustheit können die Lichtwellenleiter 44 der schwenkbaren Lichtleiter 40 miteinander und/oder ggf. mit einer in Figur 6 nicht dargestellten schwenkbaren Beleuchtungs- und Beobachtungseinrichtung verklebt, verschmolzen oder vergossen sein.

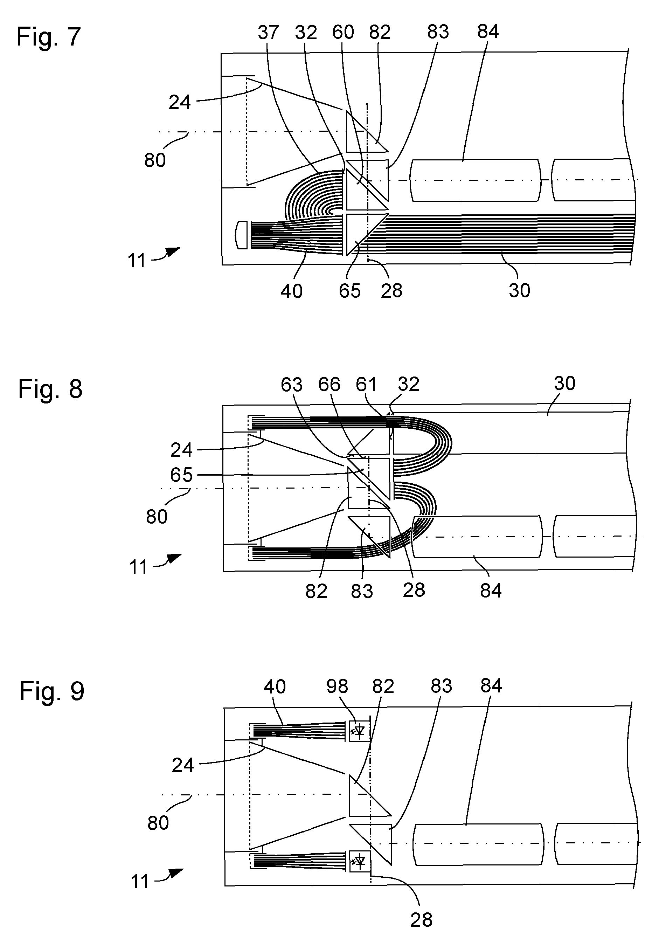

Figur 7 zeigt eine schematische Darstellung einer weiteren Ausführungsform des distalen Endes 11 des oben anhand der Figur 1 dargestellten Endoskops 10. Die Ausführungsform der Figur 7 ähnelt in einigen Merkmalen der oben anhand der Figur 6 dargestellten Ausführungsform. Insbesondere entspricht der Beobachtungsstrahlengang 80 mit einem schwenkbaren Prisma 82, einem feststehenden Prisma 83 und einer Stablinseneinrichtung 84 dem Beobachtungsstrahlengang den oben anhand der Figuren 2, 3 und 6 dargestellten Ausführungsformen.

Ferner sind bei der Ausführungsform der Figur 7 ähnlich wie bei der Ausführungsform der Figur 6 ein feststehendes Prisma 60 und ein schwenkbares Prisma 65 im Beleuchtungsstrahlengang vorgesehen. Bei der Ausführungsform der Figur 7 ist das feststehende Prisma 60 jedoch so angeordnet, dass die reflektierende Fläche des feststehenden Prismas 60 des Beleuchtungsstrahlengangs und die reflektierende Fläche des feststehenden Prismas 83 des Beobachtungsstrahlengangs benachbart zueinander und im Wesentlichen parallel angeordnet sind. Dadurch kann Bauraum eingespart werden.

Der feststehende Lichtleiter 30 weist unmittelbar lichtstromaufwärts seiner Lichtaustrittsfläche 32 eine Krümmung 37 von 180 Grad auf und ist in diesem Bereich in Figur 7 teilweise hinter dem schwenkbaren Prisma 65 verborgen. Die Lichtaustrittsfläche 32 des feststehenden Lichtleiters 30 ist mit der Lichteintrittsfläche 61 des feststehenden Prismas 60 optisch gekoppelt und kann mit dieser insbesondere verschweißt, verklebt oder mittels eines transparenten Lots oder Kitts verbunden sein.

Die Lichtaustrittsfläche 68 des schwenkbaren Prismas 65 ist mit der Lichteintrittsfläche 41 eines schwenkbaren Lichtleiters 40 optisch gekoppelt, insbesondere verschweißt, verklebt oder mittels eines transparenten Lots oder Kitts verbunden. Der schwenkbare Lichtleiter 40 umfasst insbesondere ein Bündel von miteinander verschweißten, verklebten oder vergossenen Lichtwellenleitern. Die Lichtaustrittsfläche 42 des schwenkbaren Lichtleiters 40 ist mit einer Linse 58 zur Formung des Beleuchtungslichtstrahls gekoppelt. Eine derartige Linse kann auch bei den Ausführungsformen der Figuren 2, 3, 6 und den nachfolgend beschriebenen Ausführungsformen der Figuren 8 und 9 an den Lichtaustrittsflächen 42 der schwenkbaren Lichtleiter 40 vorgesehen sein.

Bei der Ausführungsform der Figur 7 sind somit das feststehende Prisma 60 des Beleuchtungsstrahlengangs und das feststehende Prisma 83 des Beobachtungsstrahlengangs zwischen dem schwenkbaren Prisma 65 des Beleuchtungsstrahlengangs und dem schwenkbaren Prisma 82 des Beobachtungsstrahlengangs angeordnet. Wenn eine Beleuchtungs- und Beobachtungseinrichtung ähnlich der oben anhand der Figur 3 dargestellten vorgesehen ist, sind die feststehenden Prismen 60, 83 in einer Ausnehmung der Beleuchtungs- und Beobachtungseinrichtung angeordnet.

Abweichend von der Darstellung in Figur 7 können zwei oder mehr schwenkbare Lichtleiter 40 vorgesehen sein. Die Lichtaustrittsflächen der schwenkbaren Lichtleiter 40 können ähnlich wie bei den oben anhand der Figuren 2 bis 6 dargestellten Ausführungsformen am Rand 25 oder nahe dem Rand 25 des Lichtschachts 24 angeordnet sein.

Figur 8 zeigt eine schematische Darstellung einer weiteren Ausführungsform des distalen Endes 11 des oben anhand der Figur 1 dargestellten Endoskops 10. Die Ausführungsform der Figur 8 ähnelt in einigen Merkmalen den Ausführungsformen der Figuren 2, 3, 6 und insbesondere der Ausführungsform der Figur 7.

Die Ausführungsform der Figur 8 unterscheidet sich von der Ausführungsform der Figur 7 insbesondere dadurch, dass zwei schwenkbare Lichtleiter vorgesehen sind, deren Lichtaustrittsflächen 42 ähnlich wie bei den Ausführungsformen der Figuren 2, 3 und 6 in Fassungen 56 am Rand 25 des Lichtschachts 24 angeordnet sind. Ferner sind nicht die feststehenden Prismen 60, 83 des Beleuchtungsstrahlengangs und des Beobachtungsstrahlengangs, sondern die schwenkbaren Prismen 65, 82 des Beleuchtungsstrahlengangs und des Beobachtungsstrahlengangs benachbart zueinander angeordnet. Die schwenkbaren Prismen 65, 82 des Beleuchtungsstrahlengangs und des Beobachtungsstrahlengangs sind mit ihren reflektierenden Flächen benachbart und im Wesentlichen parallel zueinander angeordnet und bilden zusammen ein kompaktes, im Wesentlichen quaderförmiges schwenkbares Bauteil und/oder sind gemeinsam in einer schwenkbaren Beleuchtungs- und Beobachtungseinrichtung ähnlich der oben anhand der Figur 3 dargestellten gehalten.

Bei der Darstellung in Figur 8 ist ein schwenkbarer Lichtleiter 40 durch das feststehende Prisma 83 des Beobachtungsstrahlengangs 80 und die Stablinseneinrichtung 84 teilweise verdeckt, und ein anderer Lichtleiter 40 verdeckt teilweise den feststehenden Lichtleiter 30 und das feststehende Prisma 60 des Beleuchtungsstrahlengangs. Abweichend von der Darstellung in Figur 8 können die Lichtleiter 40 innerhalb der für die Lichtleiter 40 geltenden physikalischen Grenzen beliebig angeordnet und gekrümmt sein.

Abweichend von der Darstellung in Figur 8 kann an der Lichteintrittsfläche 66 des schwenkbaren Prismas 65 im Beleuchtungsstrahlengang oder benachbart zu dieser eine Lichtquelle, beispielsweise eine Leuchtdiode, angeordnet sein. In diesem Fall können das feststehende Prisma 60 und der feststehende Lichtleiter 30 entfallen.

Figur 9 zeigt eine schematische Darstellung einer weiteren Ausführungsform des distalen Endes 11 des oben anhand der Figur 1 dargestellten Endoskops 10. Die Ausführungsform der Figur 9 ähnelt in einigen Merkmalen den Ausführungsformen der Figuren 2, 3, 6 und 8. Die Ausführungsform der Figur 9 unterscheidet sich von diesen insbesondere dadurch, dass je eine Lichtquelle 98 mit der Lichteintrittsfläche 41 jedes schwenkbaren Lichtleiters 40 gekoppelt ist. Bei dem dargestellten Beispiel sind die Lichtleiter 40 im Wesentlichen gerade. Abweichend von dem in Figur 9 dargestellten Beispiel können die schwenkbaren Lichtleiter 40 gekrümmt sein.

Die Lichtquellen 98 sind um die Schwenkachse 28 der Beleuchtungs- und Blickrichtung schwenkbar. Die schwenkbaren Lichtleiter 40 werden deshalb beim Schwenken der Beleuchtungs- und Blickrichtung um die Schwenkachse 28 nicht verformt. Dies reduziert das Risiko von Schäden an den schwenkbaren Lichtleitern 40 und erhöht deren Lebensdauer. Alternativ sind beispielsweise lediglich parabolisch oder elliptisch geformte Reflektoren mit der Beleuchtungs- und Blickrichtung schwenkbar, in deren Brennpunkten die feststehenden Lichtquellen 98 angeordnet sind. Die Lichtquellen 98 sind beispielsweise Leuchtdioden oder fluoreszierende oder phosphoreszierende Körper, die mittels eines von einem Lichtwellenleiter übertragenen Laserstrahls angeregt werden können.

Einige Merkmale der oben anhand der Figuren 2 bis 9 dargestellten Ausführungsformen können in anderer als der dargestellten Weise kombiniert werden. Beispielsweise können bei allen Ausführungsformen an den Lichtaustrittsflächen 42 der schwenkbaren Lichtleiter 40 Linsen zur Formung des Beleuchtungslichtstrahls vorgesehen sein, wie dies bei der Ausführungsform der Figur 7 der Fall ist. Ferner kann bei jeder Ausführungsform der Figuren 6 bis 9 der Lichtschacht 24 und dessen Rand 25 sowie ggf. die Fassung oder die Fassungen 56 gemäß einer der oben anhand der Figur 4 dargestellten Varianten ausgebildet sein. Die Lichteintrittsfläche 41 des oder der schwenkbaren Lichtleiter kann bei allen Ausführungsformen gemäß einer der oben anhand der Figur 5 dargestellten Varianten ausgebildet sein.

Bei den oben anhand der Figuren dargestellten Ausführungsformen sind Blickrichtung und Beleuchtungsrichtung um eine gemeinsame Achse schwenkbar. Dazu kann insbesondere eine schwenkbare Beleuchtungs- und Beobachtungseinrichtung vorgesehen sein, wie sie oben anhand der Figur 3 dargestellt ist. Alternativ können die Blickrichtung und die Beleuchtungsrichtung um zwei verschiedene Achsen, die insbesondere parallel sind, schwenkbar sein.

Insbesondere wenn die oben anhand der Figur 3 dargestellte schwenkbare Beleuchtungs- und Beobachtungseinrichtung vorgesehen ist, sind Blickrichtung und Beleuchtungsrichtung gemeinsam schwenkbar. Dabei sind Blickrichtung und Beleuchtungsrichtung immer parallel oder unterscheiden sich immer um einen konstanten Winkel. Alternativ können die Blickrichtung und die Beleuchtungsrichtung unabhängig schwenkbar sein, oder es ist ein Winkel zwischen der Blickrichtung und der Beleuchtungsrichtung, insbesondere innerhalb vorbestimmter Grenzen, einstellbar. Dadurch kann beispielsweise bei geringer Gegenstandsweite eine vollständige Ausleuchtung des Blickfelds ermöglicht werden.

Alle Ausführungsformen und ihre Varianten zeigen, dass Lichtleiter 40, die zusammen mit ihren Lichteintrittsflächen 41 und ihren Lichtaustrittsflächen 42 um die Schwenkachse 28 der Beleuchtungs- und Blickrichtung schwenkbar sind und beim Schwenken insbesondere nicht verformt werden, bei der Gestaltung des distalen Endes 11 eines Endoskops zusätzliche Freiräume schaffen und neuartige, raumsparende und vorteilhafte Konfigurationen ermöglichen.

Die Lichtaustrittflächen 42 der schwenkbaren Lichtleiter können in der Regel unmittelbar benachbart zu einem Fensterbauteil, durch das das Beleuchtungslicht austritt, angeordnet sein. Damit bleibt das Beleuchtungslicht bis unmittelbar vor dem Fensterbauteil auf einen kleinen Querschnitt, nämlich den Querschnitt des Lichtleiters oder der Lichtleiter, gebündelt. Dies ermöglicht ein raumsparend schmales Fensterbauteil und fördert eine Miniaturisierung des Endoskops.

Die Ausführungsformen der Figuren 2 bis 4 und 6 bis 9 weisen jeweils Blenden 27 zur optischen Trennung des Beleuchtungsstrahlengangs oder der Beleuchtungsstrahlengänge vom Beobachtungsstrahlengang auf. Bei allen Ausführungsformen können die Blenden 27 jeweils an ein durchgehendes bzw. gemeinsames Fensterbauteil 20 (vgl. Figur 1) angrenzen, wobei sowohl am distalen Ende 11 des Endoskops 10 austretendes Beleuchtungslicht als auch in das distale Ende 11 des Endoskops 10 eintretendes Beobachtungslicht durch das durchgehende bzw. gemeinsame Fensterbauteil hindurchtreten. Der Raum, in dem sich Beleuchtungslicht ausbreiten kann, ist damit lediglich bis zur inneren Oberfläche des Fensterbauteils 20 vom Beobachtungsstrahlengang durch eine Wand getrennt bzw. optisch isoliert.

Alternativ können mehrere separate Fensterbauteile vorgesehen sein, wobei ein erstes Fensterbauteil lediglich im Beobachtungsstrahlengang und ein oder mehrere zweite Fensterbauteile ausschließlich im Beleuchtungsstrahlengang angeordnet sind. Das erste Fensterbauteil und das oder die zweiten Fensterbauteile sind separate Bauteile. Insbesondere ist das erste Fensterbauteil durch je eine Trennwand bzw. Blende 27 bzw. deren Randbereich getrennt, wobei die Trennwand bzw. Blende 27 nicht lichtdurchlässig ist. Die Trennwand bzw. Blende 27 ist insbesondere ausgebildet, um den Raumbereich, in dem Beleuchtungslicht sich ausbreitet, von dem Raumbereich, in dem die Kamera 80 angeordnet ist, bis zur äußeren Oberfläche des Endoskops 10 vollständig und vor allem lichtdicht voneinander zu trennen. Dadurch kann eine Einkopplung von Beleuchtungslicht, das im zweiten Fensterbauteil oder an seiner Oberfläche gestreut wird, in die Kamera 80 verhindert werden.

Bei jeder der hier beschriebenen Ausführungsformen können ein durchgehendes Fensterbauteil oder mehrere separate Fensterbauteile vorgesehen sein. Ferner kann ein durchgehendes bzw. gemeinsames Fensterbauteil mit einer Licht absorbierenden Trennschicht versehen sein, die eine unerwünschte unmittelbare Einkopplung von Beleuchtungslicht in den Beobachtungsstrahlengang verhindert. Eine solche Trennschicht kann beispielsweise durch implantierte oder auf andere Weise in das Material des Fensterbauteils lokal eingebrachte Ionen erzeugt werden.

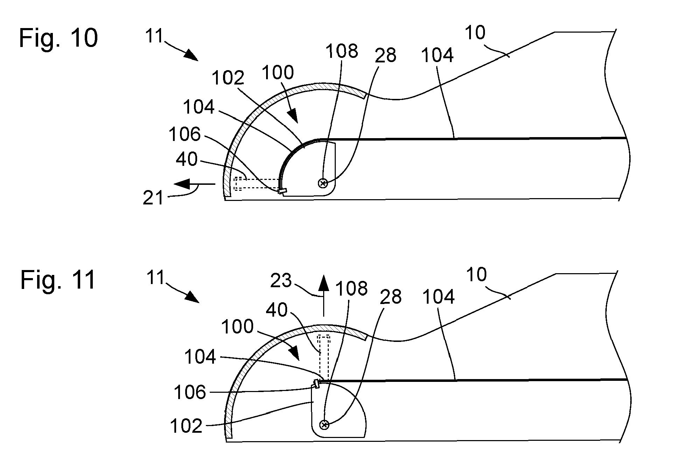

Die Figuren 10 und 11 zeigen schematische Darstellungen einer Einrichtung 100 zum Einstellen der Beleuchtungsrichtung und der Beobachtungsrichtung 21, 23 eines Endoskops 10. Die Zeichenebenen der Figuren 10 und 11 sind parallel zu den Zeichenebenen der Figuren 1 und 5, parallel zur Längsachse 18 des Endoskops 10 (vgl. Figur 1), senkrecht zur Schwenkachse 28 (vgl. Figuren 2 bis 9) und senkrecht zu den Zeichenebenen der Figuren 2 bis 4 und 6 bis 9. Das distale Ende 11 des Endoskops 10 ist in den Figuren 10 und 11 bei zwei verschiedenen Beleuchtungs- und Beobachtungsrichtungen 21, 23 dargestellt. Beispielhaft sind in gestrichelten Linien schwenkbare Lichtleiter 40 mit Fassungen nahe ihren Lichtaustrittsflächen angedeutet. Die Einrichtung 100 ist beispielsweise zum Schwenken der oben anhand der Figur 3 dargestellten bewegbaren Beleuchtungs- und Beobachtungseinrichtung 90 ausgebildet. Ferner kann die Einrichtung 100 zum Schwenken der schwenkbaren Prismen 65, 82, des Lichtschachts 24 und/oder der schwenkbaren Lichtleiter 40 von anderen der oben anhand der Figuren 2 bis 9 dargestellten Ausführungsformen ausgebildet sein.

Die Einrichtung 100 umfasst eine Scheibeneinrichtung 102, die an einer Welle 108 befestigt ist. Die Scheibeneinrichtung 102 ist mit der Welle 108 um die auch in den Figuren 1 bis 9 dargestellte Schwenkachse 28 schwenkbar. Eine Schwenkbewegung der Scheibeneinrichtung 102 hat beispielsweise eine entsprechende Schwenkbewegung der oben anhand der Figur 3 dargestellten Beleuchtungs- und Beobachtungseinrichtung 90 und der Beleuchtungs- und Beobachtungsrichtung 21, 23 zur Folge. Die Scheibeneinrichtung 102 weist einen kreisbogenförmigen Randabschnitt auf, dessen Krümmungsmittelpunkt auf der Schwenkachse 28 liegt.

Im Schaft des Endoskops 10 erstreckt sich eine Riemeneinrichtung 104 vom proximalen Ende 12 des Endoskops 10 bis zum distalen Ende 11 des Endoskops 10. Die Riemeneinrichtung 104 umfasst insbesondere einen Draht oder ein Band aus Metall oder Kunststoff und weist eine geringe Dehnungselastizität auf. Das distale Ende der Riemeneinrichtung 104 ist mittels einer Befestigungseinrichtung 106 mit der Scheibeneinrichtung 102 mechanisch verbunden.

In der Zusammenschau der Figuren 10 und 11 ist erkennbar, dass eine Bewegung der Riemeneinrichtung 104 in Längsrichtung des Schafts des Endoskops 10 eine Schwenkbewegung der Scheibeneinrichtung 102, des schwenkbaren Lichtleiters 40 und der Beleuchtungsrichtung 21, 23 bewirkt. Bei einer Schwenkbewegung der Scheibeneinrichtung 102 im Uhrzeigersinn wird ein distaler Bereich der Riemeneinrichtung 104 von dem kreisbogenförmigen Randabschnitt der Scheibeneinrichtung 102 abgehoben. Bei einer Schwenkbewegung der Scheibeneinrichtung 102 gegen den Uhrzeigersinn wird ein distaler Bereich der Riemeneinrichtung 104 auf den kreisbogenförmigen Randabschnitt aufgelegt bzw. aufgewickelt.

Die Riemeneinrichtung 104 ist insbesondere lediglich bei Zugspannung steif und kann keine Schubkräfte übertragen. Damit eine Schwenkbewegung der Scheibeneinrichtung 102, des schwenkbaren Lichtleiters 40 und der Beleuchtungs- und Beobachtungsrichtung 21, 23 gegen den Uhrzeigersinn trotzdem möglich ist, ist insbesondere eine Feder oder ein anderes elastisches Element am distalen Ende 11 des Endoskops 10 vorgesehen, das in den Figuren 10 und 11 nicht dargestellt ist. Diese Feder oder dieses andere elastische Element spannt die Scheibeneinrichtung 102 und den schwenkbaren Lichtleiter 40 in Richtung zu der in Figur 10 dargestellten Position vor.

### Bezugszeichen

- 10: Endoskop
- 11: distales Ende des Endoskops 10
- 12: proximales Ende des Endoskops 10
- 14: Schaft des Endoskops 10
- 15: erste Kupplung
- 16: zweite Kupplung
- 18: Längsachse des Endoskops 10

- 20: Fensterbauteil
- 21: erste extreme Blickrichtung (0 Grad)
- 22: zweite extreme Blickrichtung (120 Grad)
- 23: Blickrichtung (ca. 90 Grad)
- 24: Lichtschacht
- 25: Rand des Lichtschachts 24
- 27: Blende
- 28: Schwenkachse der Blickrichtung
- 29: Winkelbereich der Blickrichtungen

- 30: feststehender Lichtleiter
- 32: Lichtaustrittsfläche des feststehenden Lichtleiters 30
- 33: Lichtwellenleiter
- 35: Lichtaustrittsfläche des Lichtwellenleiters 33
- 37: Krümmung des feststehenden Lichtleiters 30 nahe seiner Lichtaustrittsfläche 32

- 40: schwenkbarer Lichtleiter
- 41: Lichteintrittsfläche des schwenkbaren Lichtleiters 40
- 42: Lichtaustrittsfläche des schwenkbaren Lichtleiters 40
- 44: Lichtwellenleiter
- 45: Lichteintrittsfläche des Lichtwellenleiters 44
- 46: Lichtaustrittsfläche des Lichtwellenleiters 44
- 48: Schwenkachse der Beleuchtungsrichtung
- 54: Krümmung des Lichtleiters 40
- 56: Fassung für Lichtwellenleiter 44 am Rand 25 des Lichtschachts 24

- 60: feststehendes Prisma
- 61: Lichteintrittsfläche des feststehenden Prismas 60
- 62: reflektierende Fläche des feststehenden Prismas 60
- 63: Lichtaustrittsfläche des feststehenden Prismas 60
- 65: schwenkbares Prisma
- 66: Lichteintrittsfläche des schwenkbaren Prismas 65
- 67: reflektierende Fläche des schwenkbaren Prismas 65
- 68: Lichtaustrittsfläche des schwenkbaren Prismas 65
- 80: Beobachtungsstrahlengang
- 82: schwenkbares Prisma
- 83: feststehendes Prisma
- 84: Stablinseneinrichtung

- 90: schwenkbare Beleuchtungs- und Beobachtungseinrichtung
- 91: erstes Lager der Beleuchtungs- und Beobachtungseinrichtung 90
- 92: zweites Lager der Beleuchtungs- und Beobachtungseinrichtung 90
- 94: Kanal für den schwenkbaren Lichtleiter 40 in der Beleuchtungs- und Beobachtungseinrichtung 90
- 96: Kanal für den feststehenden Lichtleiter 30 im ersten Lager 91
- 98: Lichtquelle
- 100: Einrichtung zum Bewegen der Lichtaustrittsfläche 32
- 102: Scheibeneinrichtung
- 104: Riemeneinrichtung
- 106: Befestigungseinrichtung zur Befestigung des distalen Endes der Riemeneinrichtung 104 an der Scheibeneinrichtung 102
- 108: Welle

## Patentansprüche

1. **Endoskop** (10) **mit einstellbarer Blickrichtung** (21, 22), mit:
einem **Lichtleiter** (40) mit einer Lichteintrittsfläche (41) und einer Lichtaustrittsfläche (42) zum Übertragen von Beleuchtungslicht von der Lichteintrittsfläche (41) zu der Lichtaustrittsfläche (42) des Lichtleiters (40),
wobei der Lichtleiter (40) mit der Lichteintrittsfläche (41) und der Lichtaustrittsfläche (42) relativ zum Endoskop (10) um eine Schwenkachse (28), die senkrecht zur Längsachse des Schafts des Endoskops ist, schwenkbar ist,
**dadurch gekennzeichnet, dass**
der schwenkbare Lichtleiter (40) ein **Bündel** von Lichtwellenleitern (44) umfasst, die miteinander verklebt, verschmolzen oder vergossen sind, um das Bündel zu versteifen,
wobei das Beleuchtungslicht mittels eines feststehenden Lichtleiters (30) von einer am proximalen Ende des Endoskops (10) angeordneten oder über ein Lichtleitkabel mit dem proximalen Ende des Endoskops gekoppelten Lichtquelle (98) zum distalen Ende des Endoskops (10) übertragen werden kann, um dort in die Lichteintrittsfläche (41) des schwenkbaren Lichtleiters (40) eingekoppelt zu werden.

2. Endoskop (10) nach dem vorangehenden Anspruch, bei dem der schwenkbare Lichtleiter (40) **starr** ist.

3. Endoskop (10) nach einem der vorangehenden Ansprüche, ferner mit:
einer schwenkbaren **reflektierenden Fläche** (62) zum Umlenken von Beleuchtungslicht aus einer Richtung parallel zur Schwenkachse (28) des schwenkbaren Lichtleiters (40) zur Lichteintrittsfläche (41) des schwenkbaren Lichtleiters (40), wobei die schwenkbare reflektierende Fläche (62) dazu vorgesehen und ausgebildet ist, um zusammen mit dem schwenkbaren Lichtleiter (40) geschwenkt zu werden.

4. Endoskop (10) nach dem vorangehenden Anspruch, bei dem die schwenkbare reflektierende Fläche (62) eine reflektierende Fläche eines schwenkbaren **Prismas** (65) mit einer Lichteintrittsfläche (61) und einer Lichtaustrittsfläche (63) ist, und die Lichteintrittsfläche (41) des schwenkbaren Lichtleiters (40) mit der Lichtaustrittsfläche (63) des schwenkbaren Prismas (65) gefügt ist.

5. Endoskop (10) nach einem der vorangehenden Ansprüche, bei dem der schwenkbare Lichtleiter (40) eine **Krümmung** (54) aufweist.

6. Endoskop (10) nach dem vorangehenden Anspruch, bei dem der schwenkbare Lichtleiter (40) an der Lichteintrittsfläche des schwenkbaren Lichtleiters (40) in einer Richtung **parallel zur Schwenkachse** (28) des schwenkbaren Lichtleiters (40) angeordnet ist.

7. Endoskop (10) nach dem vorangehenden Anspruch, bei dem die Lichteintrittsfläche (41) des schwenkbaren Lichtleiters (40) **kreisförmig** oder **kreisringförmig** ist.

8. Endoskop (10) nach einem der vorangehenden Ansprüche, ferner mit:
einem **feststehenden Lichtleiter** (30), zum Übertragen von Beleuchtungslicht zum distalen Ende (11) des Endoskops (10), wobei der feststehende Lichtleiter (30) nahe seiner Lichtaustrittsfläche (32) eine Krümmung (37) aufweist.

9. Endoskop (10) nach dem vorangehenden Anspruch, ferner mit:
einem um die Schwenkachse (28) der Blickrichtung (21, 22) schwenkbaren **Lichtschacht** (24) zur optischen Trennung des Beobachtungsstrahlengangs (80) vom Beleuchtungsstrahlengang (30, 40),
wobei die **Lichtaustrittsfläche** (42) des schwenkbaren Lichtleiters außen an dem Lichtschacht (24) angeordnet ist.

## Claims

1. Endoscope (10) with an adjustable viewing direction (21, 22), comprising:
an optical waveguide (40) with a light-entry surface (41) and a light-exit surface (42) for transmitting illumination light from the light-entry surface (41) to the light-exit surface (42) of the optical waveguide (40),
wherein the optical waveguide (40) with the light-entry surface (41) and the light exit surface (42) is swivelable relative to the endoscope (10) about a swivel axis (28) which is perpendicular to the longitudinal axis of the shank of the endoscope,
**characterized in that**
the swivelable optical waveguide (40) comprises a bundle of optical waveguides (44), which are adhesively bonded, fused or cast together in order to stiffen the bundle,
wherein the illumination light can be transmitted by means of a fixed optical waveguide (30) from a light source (98) which is arranged at the proximal end of the endoscope (10) or coupled to the proximal end of the endoscope by way of a light guiding cable, to the distal end of the endoscope (10) in order to be coupled there into the light-entry surface (41) of the swivelable optical waveguide (40).

2. Endoscope (10) according to the preceding claim, wherein the swivelable optical waveguide (40) is rigid.

3. Endoscope (10) according to one of the preceding claims, furthermore comprising:
a swivelable reflecting surface (62) for deflecting illumination light from a direction parallel to the swivel axis (28) of the swivelable optical waveguide (40) to the light-entry surface (41) of the swivelable optical waveguide (40), wherein the swivelable reflecting surface (62) is provided and embodied to be swivelled together with the swivelable optical waveguide (40).

4. Endoscope (10) according to the preceding claim, wherein the swivelable reflecting surface (62) is a reflecting surface of a swivelable prism (65) with a light-entry surface (61) and a light-exit surface (63), and the light-entry surface (41) of the swivelable optical waveguide (40) is joined to the light-exit surface (63) of the swivelable prism (65).

5. Endoscope (10) according to one of the preceding claims, wherein the swivelable optical waveguide (40) has a curve (54).

6. Endoscope (10) according to the preceding claim, wherein the swivelable optical waveguide (40) is arranged in a direction parallel to the swivel axis (28) of the swivelable optical waveguide (40) at the light-entry surface of the swivelable optical waveguide (40).

7. Endoscope (10) according to the preceding claim, wherein the light-entry surface (41) of the swivelable optical waveguide (40) is circular or circular ring-shaped.

8. Endoscope (10) according to one of the preceding claims, furthermore comprising:
a fixed optical waveguide (30) for transmitting illumination light to the distal end (11) of the endoscope (10), wherein the fixed optical waveguide (30) has a curve (37) close to the light-exit surface (32) thereof.

9. Endoscope (10) according to the preceding claim, furthermore comprising:
a light duct (24) swivelable about the swivel axis (28) of the viewing direction (21, 22) for optically separating the observation beam path (80)' from the illumination beam path (30, 40), wherein the light-exit surface (42) of the swivelable optical waveguide is arranged outside on the light duct (24).

## Revendications

1. Endoscope (10) à direction de visée (21, 22) réglable, comprenant :
un guide de lumière (40) pourvu d'une surface d'entrée de lumière (41) et d'une surface de sortie de lumière (42) servant à la transmission de lumière d'éclairage de la surface d'entrée de lumière (41) vers la surface de sortie de lumière (42) du guide de lumière (40),
le guide de lumière (40) avec la surface d'entrée de lumière (41) et la surface de sortie de lumière (42) pouvant être pivoté par rapport à l'endoscope (10) autour d'un axe de pivotement (28) qui est perpendiculaire à l'axe longitudinal de la tige de l'endoscope,
**caractérisé en ce que**
le guide de lumière pivotant (40) comprend un faisceau de fibres optiques (44) qui sont collées, soudées ou scellées ensemble afin de rigidifier le faisceau,
la lumière d'éclairage pouvant être transmise par l'intermédiaire d'un guide de lumière fixe (30) d'une source de lumière (98), disposée à l'extrémité proximale de l'endoscope (10) ou couplée par le biais d'un câble à fibres optiques avec l'extrémité proximale de l'endoscope, à l'extrémité distale de l'endoscope (10) pour y être injectée dans la surface d'entrée de lumière (41) du guide de lumière pivotant (40).

2. Endoscope (10) selon la revendication précédente, dans lequel le guide de lumière pivotant (40) est rigide.

3. Endoscope (10) selon l'une des revendications précédentes, comprenant en outre :
une surface réfléchissante (62) pivotante destinée à dévier la lumière d'éclairage d'une direction parallèle à l'axe de pivotement (28) du guide de lumière pivotant (40) vers la surface d'entrée de lumière (41) du guide de lumière pivotant (40), la surface réfléchissante (62) pivotante étant conçue et configurée pour être pivotée conjointement avec le guide de lumière pivotant (40).

4. Endoscope (10) selon l'une des revendications précédentes, dans lequel la surface réfléchissante (62) pivotante est une surface réfléchissante d'un prisme (65) pivotant pourvu d'une surface d'entrée de lumière (61) et d'une surface de sortie de lumière (63), et la surface d'entrée de lumière (41) du guide de lumière pivotant (40) étant jointe à la surface de sortie de lumière (63) du prisme (65) pivotant.

5. Endoscope (10) selon l'une des revendications précédentes, dans lequel le guide de lumière pivotant (40) possède une courbure (54).

6. Endoscope (10) selon la revendication précédente, dans lequel le guide de lumière pivotant (40), au niveau de la surface d'entrée de lumière du guide de lumière pivotant (40), est disposé dans une direction parallèle à l'axe de pivotement (28) du guide de lumière pivotant (40).

7. Endoscope (10) selon la revendication précédente, dans lequel la surface d'entrée de lumière (41) du guide de lumière pivotant (40) est de forme circulaire ou torique.

8. Endoscope (10) selon l'une des revendications précédentes, comprenant en outre :
un guide de lumière fixe (30) servant à la transmission de la lumière d'éclairage vers l'extrémité distale (11) de l'endoscope (10), le guide de lumière fixe (30) possédant une courbure (37) à proximité de sa surface de sortie de lumière (32).

9. Endoscope (10) selon la revendication précédente, comprenant en outre :
un puits de lumière (24) pivotant autour de l'axe de pivotement (28) de la direction de visée (21, 22) servant la séparation optique du trajet de rayon d'observation (80) du trajet de rayon d'éclairage (30, 40),
la surface de sortie de lumière (42) du guide de lumière pivotant étant disposée à l'extérieur sur le puits de lumière (24).
